Europäisches Patentamt

European Patent Office

Office européen des brevets

(11) Veröffentlichungsnummer: **0 283 977 B1**

(12) **EUROPÄISCHE PATENTSCHRIFT**

(45) Veröffentlichungstag der Patentschrift: 31.07.91

(51) Int. Cl.5: **A61K 47/44, A61K 9/02**

(21) Anmeldenummer: 88104433.3

(22) Anmeldetag: 19.03.88

(54) **Emulgierende Suppositoriengrundmassen und daraus hergestellte Zäpfchen.**

(30) Priorität: 25.03.87 DE 3709861

(43) Veröffentlichungstag der Anmeldung:
28.09.88 Patentblatt 88/39

(45) Bekanntmachung des Hinweises auf die
Patenterteilung:
31.07.91 Patentblatt 91/31

(84) Benannte Vertragsstaaten:
CH DE ES FR IT LI

(56) Entgegenhaltungen:
LU-A- 82 760

CHEMICAL ABSTRACTS, Band 88, Nr. 22, 29.
Mai 1978, Seite 387, Zusammenfassung Nr.
158485p, Columbus, Ohio, US; & JP-A-77 156
918 (SUMITOMO CHEMICAL CO., LTD)
27-12-1977

CHEMICAL ABSTRACTS, Band 98, Nr. 26, 27.
Juni 1983, Seite 376, Zusammenfassung Nr.
221748a, Columbus, Ohio, US; Y. NISHIOKA
et al.: "Rectal administration of insulin suppositories. 4. The influences of water content and surfactant concentration in insulin
soft capsule suppository base on its hypoglycemic activity" & YAKUZAIGAKU 1982,

42(4), 287-93

CHEMICAL ABSTRACTS, Band 105, Nr. 6, 11.
August 1986, Seite 387, Zusammenfassung
Nr. 48957f, Columbus, Ohio, US; H. YOSHIKA-
WA et al.: "Development of interferon suppositories. I. Enhanced rectal absorption of
human fibroblast interferon by fusogenic lipid via lymphotropic delivery in rats",

PHARMACEUTICAL RESEARCH, vol. 3, no.2,
1986, pages 116-117

(73) Patentinhaber: Henkel Kommanditgesellschaft
auf Aktien
Postfach 1100 Henkelstrasse 67
W-4000 Düsseldorf-Holthausen(DE)

(72) Erfinder: Breitzke, Willi
Am Falder 20
W-4000 Düsseldorf 13(DE)

**Beschreibung**

Die Erfindung betrifft Suppositoriengrundmassen mit guter Emulgierwirkung für Wasser, wäßrige Wirkstofflösungen und polare Wirkstoffe.

Suppositorien sind zylindrisch oder leicht oval geformte Zäpfchen aus einer bei Körpertemperatur schmelzfähigen Masse, in der Arzneistoffe gelöst, dispergiert oder emulgiert sind und die zur Applikation dieser Arzneistoffe in den Enddarm des Menschen bestimmt sind.

Suppositorien setzen sich daher im wesentlichen aus der Suppositoriengrundmasse und den Wirkstoffen zusammen. Die Suppositoriengrundmasse kann gewisse Hilfsstoffe enthalten, welche das Schmelzverhalten, die Viskosität, die Verarbeitbarkeit und die Stabilität der Massen und der daraus hergestellten Zäpfchen verbessern oder die der Verbesserung der Verteilung der Wirkstoffe in Zäpfchen und der Resorption der Wirkstoffe im Rektum dienen. Als Suppositoriengrundmassen werden vorwiegend wasserunlösliche Fettmassen eingesetzt. Man kennt auch wasserlösliche Massen auf der Basis von Polyäthylenglycolen, die jedoch wegen der Unverträglichkeit mit vielen Wirkstoffen, der Hygroskopizität und dadurch ausgelöste Schleimhautirritationen und der Tendenz zu Nachhärtungen nur eine begrenzte Bedeutung besitzen. Schließlich sind auch wasserdispergierbare Massen auf Basis bestimmter nichtionogener oberflächenaktiver Ethylenoxidaddukte bekannt, diese haben aus ähnlichen Gründen keine wirtschaftliche Bedeutung erlangt. Eine gewisse Bedeutung haben aber die "hydrophilen Fettgrundmassen" erlangt, die Gemische aus lipophilen Fettmassen und bestimmten nichtionogenen Emulgatoren darstellen.

Aus Chemical Abstracts, Band 88, (1978), Seite 387), Nr. 158485p waren Penicillinpräparate für die rektale Applikation bekannt, die Penicilline in einem Träger aus Erdnußöl und einem nichtionogenen Tensid enthalten. Aus Chemical Abstracts, Band 98, (1983), Seite 376, Nr. 221748a waren Weichgelatine-Kapsel-Suppositorien bekannt, die Insulin in einem Träger aus einer Olivenöl-Emulsion mit einem oxethylierten, hydrierten Rizinusöl als Emulgator enthalten. Aus Pharm. Res. 1986, 3 (2), 116 - 117 (H. Yoshikawa et al.) waren Interferon-Suppositorien bekannt, die den Wirkstoff in einem Träger aus einer Fettmasse und Hilfsstoffen, wie z.B. einem Anlagerungsprodukt von 60 Mol Ethylenoxid an hydriertes Rizinusöl und/oder Linolsäure enthalten.

Die bekannten Suppositoriengrundmassen auf Basis von lipophilen Fettmassen und auch die bekannten "hydrophilen" Suppositoriengrundmassen sind jedoch nicht geeignet, wäßrige Wirkstoffzubereitungen oder stark polare Wirkstoffe wie z. B. Phenole, Glycerin, Zucker in den erforderlichen, oft recht großen Mengen problemlos und stabil in den Zäpfchen zu verteilen. Es treten vielmehr häufig Separationen in den gegossenen Suppositorien auf. Als Folge davon werden inhomogene, bröckelige oder wenig bruchfeste Zäpfchen erhalten, die zu erheblichen Problemen bei der Produktion oder bei der Anwendung führen.

Es bestand daher die Aufgabe, Suppositoriengrundmassen auf Basis wasserunlöslicher Fettmassen zu finden, in welche sich auch verdünnte wäßrige Lösungen oder Dispersionen bestimmter Wirkstoffe und andere stark polare Komponenten wie z.B. Glycerin, Phenole, Salze, z.B. von Schieferölsulfonsäuren (Ichthyol [R]) in größeren Mengen problemlos, homogen und stabil einemulgieren lassen.

Die Aufgabe wird erfindungsgemäß gelöst durch Suppositoriengrundmassen, bestehend aus einer wasserunlöslichen Fettmasse und einem Emulgator, dadurch gekennzeichnet, daß als Emulgator ein Anlagerungsprodukt von 5 - 50 Mol Ethylenoxid an 1 Mol gehärtetes Rizinusöl enthalten ist.

Als wasserunlösliche Fettmassen werden dabei solche Fettmassen verstanden, wie sie in der Monographie "Suppositorien" von Prof. Dr. Bernd W. Müller (Wisschenschaftliche Verlagsgesellschaft mbH, Stuttgart, 1986) in Kapitel 2.2.4.2 auf Seiten 94 - 101 näher beschrieben sind. Es handelt sich dabei um Kakaobutter, gehärtete Pflanzenöle und vor allem um Gemische von Mono-, Di- und Triglyceriden der gesättigten Fettsäuren $C_{10}H_{20}O_2$ bis $C_{18}H_{36}O_2$, die der Spezifikation nach DAB 9 (1986) und dem Europäischen Arzneibuch für Hartfett (Adeps solidus) entsprechen. In Ausnahmefällen werden auch Fettsäureester einwertiger Alkohole (Wachse) oder zweiwertiger Alkohole (z. B. 1.2- Propylenglycoldimyristat oder -palmitat), verzweigte Fettalkohole (Guerbetalkohole mit 32 - 40 C-Atomen) oder Dicetylphthalat als wasserunlösliche Suppositorien-Fettmassen verwendet.

Die weitaus größte Bedeutung kommt jedoch den Glycerin-Fettsäure-Estern (Mono-, Di-, und Triglyceride) zu. Diese werden entweder aus gehärteten natürlichen Fetten, z. B. Kokosöl oder Plamkernfett durch Umesterung mit Glycerin und gegebenenfalls Fettsäuren anderer C-Kettenverteilung zur Einstellung der gewünschten Kenndaten (Säurezahl und Hydroxylzahl) und des gewünschten Schmelzverhaltens hergestellt. Man kann aber auch geeignete Fettsäureschnitte, bevorzugt mit 10 - 18 C-Atomen direkt mit Glycerin verestern.

Das als Emulgator geeignete Anlagerungsprodukt von 5 - 50 Mol Ethylenoxid an 1 Mol gehärtetes Rizinusöl wird durch Oxethylierung von gehärtetem Rizinusöl unter an sich bekannten Bedingungen hergestellt, d. h. das gehärtete Rizinusöl wird in Gegenwart von basischen Katalysatoren, wie z. B.

Natriummethylat oder Kaliumhydroxid mit der berechneten Menge Ethylenoxid bei Temperaturen von 140 °C - 200 °C umgesetzt.

Die erfindungsgemäßen Suppositoriengrundmassen können 0,1 bis 20 Gew.-% des Emulgators enthalten. Daneben können die erfindungsgemäßen Suppositoriengrundmassen in geringerem Umfang, etwa bis insgesamt 5 Gew.-% andere, für die Regulierung des Schmelzverhaltens, der Viskosität und Verarbeitbarkeit und zur Erhöhung der Lagerstabilität und der Verbesserung des Aussehens der Suppositorienmasse oder der daraus herzustellenden Zäpfchen bekannte Hilfsstoffe wie z. B. Bienenwachs, Cetylstearylalkohol, Glycerinmonostearat, Aluminiumstearat, Bentonit, Cellulosen, flüssige Triglyceride, Paraffine, hochdisperse Kieselsäure bzw. Kieselgur, Calciumcarbonat, Magnesiumoxid, Antioxidantien (z. B. Tocopherole, Butylhydroxytoluol, Ascorbylpalmitat, Propylgallat), Konservierungsmittel und Farbstoffe enthalten.

In einer bevorzugten Ausführungsform bestehen die erfindungsgemäßen Suppositoriengrundmassen im wesentlichen aus 90 - 99,8 Gew.-% einer Glycerin-Fettsäureester-Masse und 0,2 - 10 Gew.-% eines Anlagerungsproduktes von 5 - 50 Mol Ethylenoxid an 1 Mol gehärtetes Rizinusöl.

Weiterhin sind besonders solche erfindungsgemäßen Suppositoriengrundmassen besonders bevorzugt, die einen Steigschmelzpunkt von 29 - 45 °C aufweisen.

Die erfindungsgemäßen Suppositoriengrundmassen sind besonders gut geeignet zur Herstellung wasserhaltiger Suppositorien, d. h. zur Einarbeitung von Wirkstoffen, die nur in wäßriger Lösung verfügbar oder verarbeitbar sind. Solche Wirkstoffe können in solchen Mengen mit den erfindungsgemäßen Suppositoriengrundmassen verarbeitet werden, daß die dabei erhaltenen Zäpfchen bis zu 30 Gew.-% Wasser homogen verteilt, d. h. homogen dispergiert enthalten.

Ein weiterer Gegenstand der Erfindung sind daher wasserhaltige Suppositorien, dadruch gekennzeichnet, daß sie 70 - 99,5 Gew.-% der erfindungsgemäßen Suppositoriengrundmasse und 0,5 -30 Gew.-% darin homogen dispergiertes Wasser enthalten. Solche Suppositorien zeichnen sich durch gute Homogenität, Bruchfestigkeit und durch eine gute Schleimhautverträglichkeit aus. Die folgenden Beispiele sollen den Erfindungsgegenstand näher erläutern.

## Beispiele

1. Herstellung erfindungsgemäßer Suppositoriengrundmassen.

| | | |
|---|---|---|
| 1.1 | Novata<sup>(R)</sup> BC | 987,8 g |
| | Hydriertes Rizinusöl + 40 Mol EO | 12,2 g |
| | | 1000,0 g |

Die Bestandteile werden gemeinsam bei ca. 50 °C aufgeschmolzen und homogenisiert. Nach dem Abkühlen auf 20 °C wurden homogene Massen mit einem Steigschmelzpunkt von 34,4 °C erhalten.

| | | |
|---|---|---|
| 1.2 | Novata<sup>(R)</sup>BC | 951,2 g |
| | Hydriertes Rizinusöl + 7 Mol EO | 48,8 g |
| | | 1000,0 g |

Die Herstellung erfolgte analog Beispiel 1. Es wurde eine homogene Masse mit einem Steigschmelzpunkt von 34,2 °C erhalten.

|       |                                   |            |
|-------|-----------------------------------|------------|
| 1.3   | Novata[R] A                       | 977,8 g    |
|       | Hydriertes Rizinusöl + 7 Mol EO   | 22,2 g     |
|       |                                   | 1000,0 g   |

Die Herstellung erfolgte analog Beispiel 1. Es wurde eine homogene Masse mit einem Steigschmelzpunkt von 34,2 °C erhalten.

Es wurden folgende Handelsprodukte verwendet:

Novata [R] (Henkel KGaA, Düsseldorf) und

Novata BC

Gemische von Triglyceriden, mit geringen Anteilen an Di- und Monoglyceriden, eines $C_{12}$-$C_{18}$-Fettsäureschnitts.

| Kenndaten        | Novata A        | Novata BC         |
|------------------|-----------------|-------------------|
| Steigschmelzpunkt | 33,5 - 35,5 °C | 33,5 - 35,5 °C   |
| Erstarrungspunkt | 29 - 31 °C      | 30,5 - 32,5 °C   |
| Säurezahl        | unter 0,3       | unter 0,3         |
| Verseifungszahl  | 225 - 240       | 225 - 240         |
| Hydroxylzahl     | 35 - 45         | 30 - 40           |

2. Herstellung von Suppositorien

|      |                                          |        |
|------|------------------------------------------|--------|
| 2.1  | Suppositoriengrundmasse Beispiel 1.1     | 82 g   |
|      | Wäßrige Suspension der korpuskulären     |        |
|      | Bestandteile und Stoffwechselproduk-     |        |
|      | te von 1700 Mio. Escherichia Coli und    |        |
|      | 17 mg Phenol pro g Suspension            | 18 g   |
|      |                                          | 100 g  |

Herstellung: Die Suppositorienmasse wurde bei 50 °C aufgeschmolzen. Nach dem Abkühlen auf 38 °C wurde die Wirkstoff-Suspension zugegeben und homogen in der Schmelze verteilt. Nach Abkühlung auf ca. 33 °C wurde die homogene Dispersion in die Zäpfchenform gegossen und bei 20 °C nach 1 Stunde entformt. Es wurden homogene Zäpfchen mit einem Gewicht von ca. 2,15 g erhalten.

2.2 Suppositoriengrundmasse nach Beispiel 1.2   82 g

Wäßrige Suspension gemäß Beispiel 2.1   18 g

———————

100 g

Die Herstellung der Suppositorien erfolgte analog Beispiel 2.1.

2.3 Suppositoriengrundmasse nach Beispiel 1.3   82,0 g

Glycerin 86 %ig   15,0 g

Milchzucker   1,5 g

Extractum Belladonna   1,5 g

———————

100,0 g

Herstellung:   Die Suppositorienmasse wurde bei 50 °C aufgeschmolzen. Nach dem Abkühlen auf 38 °C wurden Glycerin, Milchzucker und Extractum Belladonna zugesetzt und homogen in der Schmelze verteilt. Nach Abkühlung auf 33 °C wurde die homogene Dispersion in die Zäpfchenform gegossen und bei 20 °C nach 1 Stunde entformt. Es wurden homogene Zäpfchen von ca. 2,15 g Gewicht erhalten.

**Patentansprüche**

1. Suppositoriengrundmassen bestehend aus einer wasserunlöslichen Fettmasse und einem Emulgator, dadurch gekennzeichnet, daß als Emulgator ein Anlagerungsprodukt von 5 - 50 Mol Ethylenoxid an 1 Mol gehärtetes Rizinusöl enthalten ist.

2. Suppositoriengrundmasse gemäß Anspruch 1, dadurch gekennzeichnet, daß sie im wesentlichen aus 90 -99,8 Gew.-% einer Glycerin-Fettsäureester-Masse und 0,2 - 10 Gew.-% eines Anlagerungsproduktes von 5 - 50 Mol Ethylenoxid an 1 Mol gehärtetes Rizinusöl besteht.

3. Suppositoriengrundmasse gemäß Anspruch 1 oder 2, dadurch gekennzeichnet, daß sie einen Steigschmelzpunkt von 29 - 45 °C aufweist.

4. Wasserhaltige Suppositorien, dadurch gekennzeichnet, daß sie 70 - 99,5 Gew.-% der Suppositoriengrundmasse gemäß Anspruch 1 - 3 und 0,5 - 30 Gew.-% darin homogen dispergiertes Wasser enthalten.

**Claims**

1. Compositions for suppository bases consisting of a water-insoluble fat and an emulsifier, characterized in that an adduct of 5 to 50 mol ethylene oxide with 1 mol hydrogenated castor oil is present as the emulsifier.

2. A composition for suppository bases as claimed in claim 1, characterized in that it consists essentially of 90 to 99.8% by weight of a glycerol fatty acid ester material and 0.2 to 10% by weight of an adduct of 5 to 50 mol ethylene oxide with 1 mol hydrogenated castor oil.

3. A composition for suppository bases as claimed in claim 1 or 2, characterized in that it has an elevation

EP 0 283 977 B1

melting point of 29 to 45° C.

4. Water-containing suppositories, characterized in that they contain 70 to 99.5% by weight of the suppository base claimed in claims 1 to 3 and 0.5 to 30% by weight water homogeneously dispersed therein.

**Revendications**

1. Matière de base pour suppositoires qui consistent en une masse grasse insoluble dans l'eau et en un agent émulsionnant, caractérisées en ce qu'en tant qu'agent émulsionnant, elles contiennent un produit d'addition de 5 à 50 ml d'oxyde d'éthylène sur 1 mol d'huile de ricin durcie.

2. Matière de base pour suppositoires selon la revendication 1, caractérisée en ce qu'elle consiste pour l'essentiel en 90 à 99,8 % en poids d'une masse d'ester glycérique d'acide gras et de 0,2 à 10 % en poids d'un produit d'addition de 5 à 50 moles d'oxyde d'éthylène sur 1 mole d'huile de ricin durcie.

3. Matière de base pour suppositoires selon la revendication 1 ou 2, caractérisée en ce qu'elle possède un point de fusion montant de 29 à 45° C.

4. Suppositoires, contenant de l'eau caractérisés en ce qu'ils renferment 70 à 99,5 % en poids de la masse pour suppositoire selon les revendications 1 à 3 et 0,5 à 30 % en poids d'eau dispersée d'une manière homogène dans celle-ci.

6